# EUROPEAN PATENT SPECIFICATION

(11) **EP 0 422 083 B1**
(45) Date of publication and mention of the grant of the patent: **21.12.1994**
(21) Application number: 89907636.8
(22) Date of filing: 28.06.1989
(51) Int. Cl.: A61M 5/00, A61M 5/32, B26D 1/28, A61B 19/02, B02C 18/14

(54) **SHARPS DESTRUCTION AND DISPOSAL APPARATUS**
VERNICHTUNG UND ABFALL VON SCHARFEN GEGENSTÄNDEN
APPAREIL DE DESTRUCTION ET DE MISE AU REBUT D'OBJETS TRANCHANTS

(30) Priority: 28.06.1988 AU 9032/88; 14.11.1988 AU 1452/88
(43) Date of publication of application: 17.04.1991
(73) Proprietor: ELOTOWN PTY. LTD., Port Macquarie, NSW 2444 (AU)
(72) Inventor: McCARTHY, Ronald James, Port Macquarie, NSW 2444 (AU)
(74) Representative: Chettle, Adrian John
(86) International application number: AU8900277
(87) International publication number: WO9000070

(56) References cited:
- EP-A- 80 882
- EP-A- 257 925
- EP-A- 267 776
- AU-A- 8 238 687
- AU-B- 3 638 458
- GB-A- 1 302 851
- US-A- 3 995 768
- US-A- 4 580 688
- US-A- 4 657 139

## Description

### TECHNICAL FIELD

The present invention relates to the safe disposal of hypodermic syringes, needles and other small, contaminated instruments such as, for example, scalpels blades, stitch cutters, etc. (commonly referred to as "sharps").

### BACKGROUND ART

It is necessary to safely dispose of contaminated medical tools and other materials used in the medical environment. Such tools and the materials (ie. sharps), or more specifically disposable percutaneous devices, have been used, they must be safely disposed of so as to avoid reuse or accidental physical contact (eg. needle stick injury).

One present known method of disposing of the above mentioned contaminated materials is to place the used syringe and needle into an apparatus which merely cuts the needle (sharp) from the syringe. The needle is kept in a container portion of the apparatus for later disposal, and the syringe portion is disposed of separately.

Alternatively, in large hospitals, medical centres and pathology departments, the whole needle (sharp) and syringe is placed in any one of a variety of containers as a complete unit, along with other contaminated medical tools or instruments. These containers are then disposed of by either incineration or burial at local garbage tips. An example of such a container is disclosed in US-A-4580688, in which the container has an upper entry chute through which contaminated waste is directed into the container, and a door which closes the lower end of the chute when the container is full before it is buried or otherwise disposed of.

From EP-A-80882 a reusable sharp container for receiving sharps according to the preamble of claim 1 is known in which the container is operated by a pedal for opening and closing a pivotable lid to enable sharps to be diposited inside.

EP-A-267776 discloses an sharp container with a first lid including a second lid. The first lid is however unreleasable connected with the container. Furthermore EP-A-267776 discloses a method for disposing of contaminated sharps such as defined in the preamble of claim 21.

Incineration of complete syringe is difficult and often incomplete after considerable incineration time. Further, the sharps and other instruments normally collected for disposal are usually (70-80%) highly infectious, and may remain infectious for a considerable time even after burial.

The first mentioned known method of disposal is awkward and requires the person disposing of the syringe to dispose of the syringe portions separately to that of the needle (sharp). The second above mentioned method of disposal requires the complete syringe and needle unit to be disposed of in one piece. Syringe and needle units are relatively bulky, and when a large number require disposal, the volume thereof can be inconveniently large.

Accordingly, in US-A-3995768 there is disclosed an apparatus for destroying medical syringes by directing the syringes to drop one by one between two serrated grinding surfaces, one of which rotates. The serrations bite into the syringe which is thereby crushed or ground between them. This grinding action is susceptable to jamming and, of course, can only destroy syringes singly.

### OBJECTS OF INVENTION

It is an object of the present invention to provide a container which enables convenient collection of the sharps and a means of transferring the sharps from the container into a sharps destruction apparatus which comminutes and sterilizes the sharps into a generally harmless state suitable for easy disposal by incineration or burial.

### DISCLOSURE OF THE INVENTION

In one broad form the present invention provides a reusable sharps container as defined in claim 1 having a primary opening and a primary lid adapted to close the primary opening in said container, said primary lid being movable with respect to the container between a closed position closing the primary opening, and an open position opening the primary opening;
said container further having a secondary opening and a secondary lid adapted to close the secondary opening, said secondary lid being movable between a closed position closing the secondary opening, and an open position opening the secondary opening;
said container further comprising a latch means adapted to retain the primary lid in the closed position, and to automatically open the primary lid when said container is brought into cooperation with a disposal receptacle of an apparatus which is adapted to operate on said latch means,
wherein said sharps are inserted into the container through said secondary opening but are removed through the primary opening directly into said disposal receptacle.

Another broad form of the present invention provides a method of disposing of contaminated medical tools, sharps and other materials, as defined in claim 21, comprising:
closing a primary opening in a reusable container with a primary lid which is secured in a closed position by a latch means;
collecting said materials in said reusable container by inserting same through a secondary opening in said container, the secondary opening being closable by a secondary lid;
inverting and inserting said reusable container in a receptacle of an apparatus which is adapted to automatically release said latch means, whereby said primary lid opens and said material is discharged directly into said receptacle of said apparatus; and
removing the reusable container from the receptacle for reuse.

### BRIEF DESCRIPTION OF THE DRAWINGS

Preferred forms of the present invention will now be described by way of example with reference to the accompanying drawings, wherein:
Figure 1 is a schematic cross-sectional view of the sharps destructor apparatus.
Figure 2 is a longitudinal side elevation sectional view of the apparatus of Figure 1.
Figure 3 is a schematic longitudinal sectional top plan view of the apparatus of Figure 1.
Figure 4 is a longitudinal sectional view of the rotating cutter means of the destructor apparatus.
Figure 5 is a top plan view of a cutter blade of the destructor apparatus.
Figure 6 is a top plan view of the upper cutter plate of the destructor apparatus.
Figure 7 is a top plan view of the lower cutter plate of the destructor apparatus.
Figure 8 is a schematic perspective exploded view of the sharps collection bin.
Figure 9 is a cross-sectional view of the lid portion of the collection bin.
Figure 10 is a schematic perspective view of the housing showing the disposal receptacle and washing receptacle.

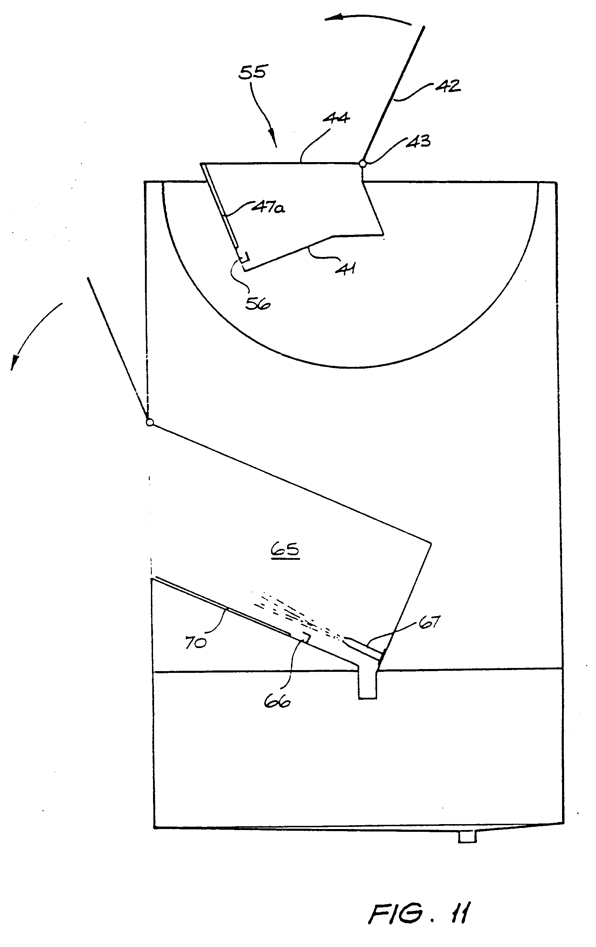

Figure 11 is a cross sectional view of the housing of Figure 10.

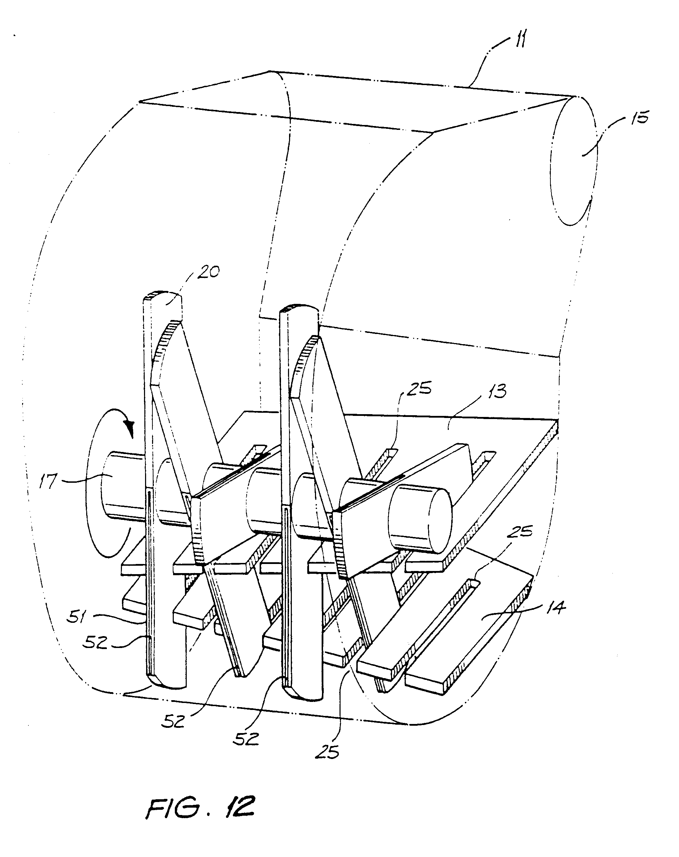

Figure 12 is a schematic perspective view of the destructor chamber.

### BEST MODE FOR CARRYING OUT THE INVENTION

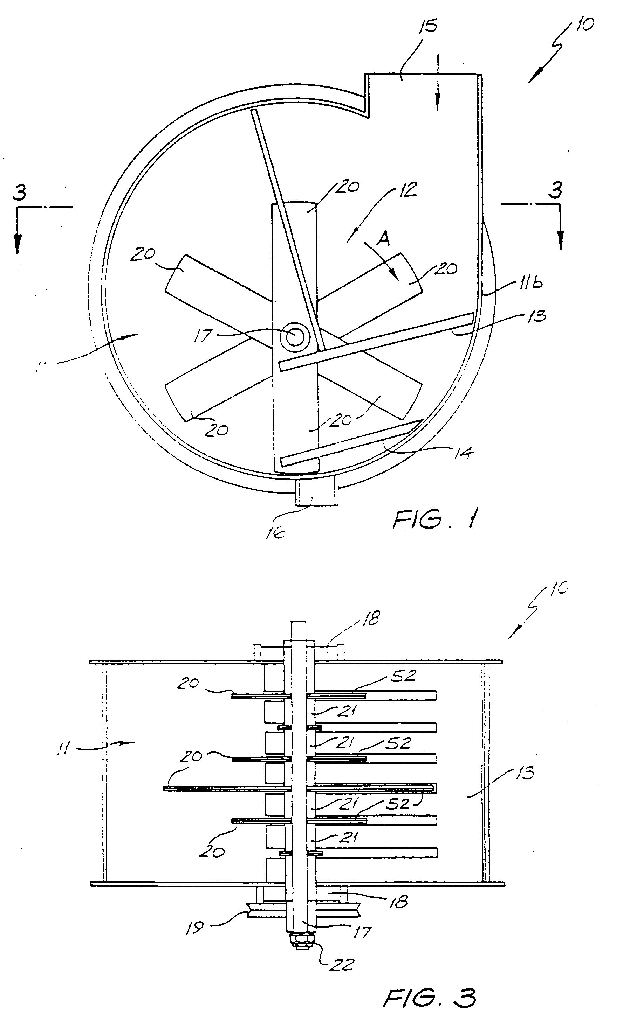

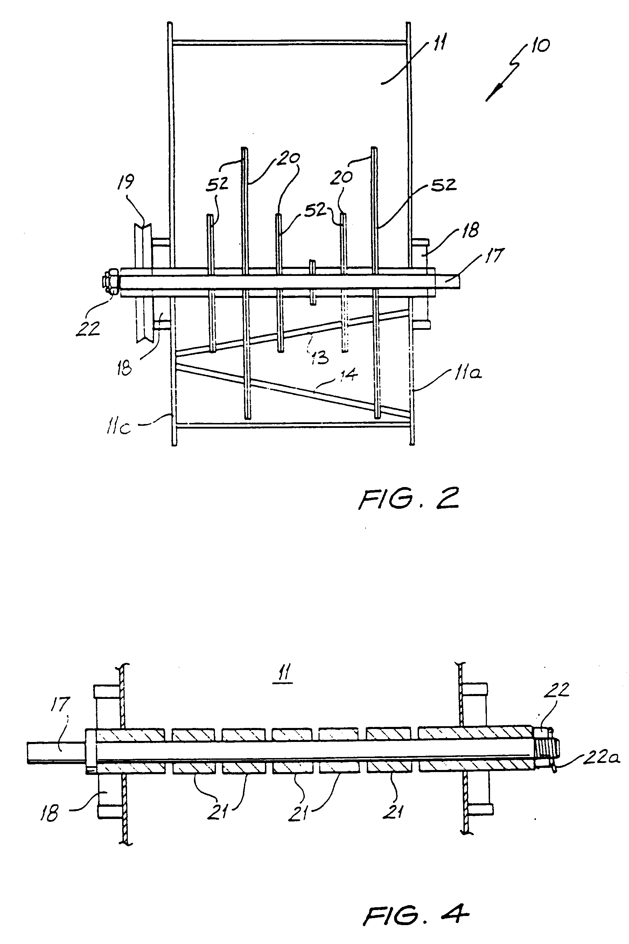

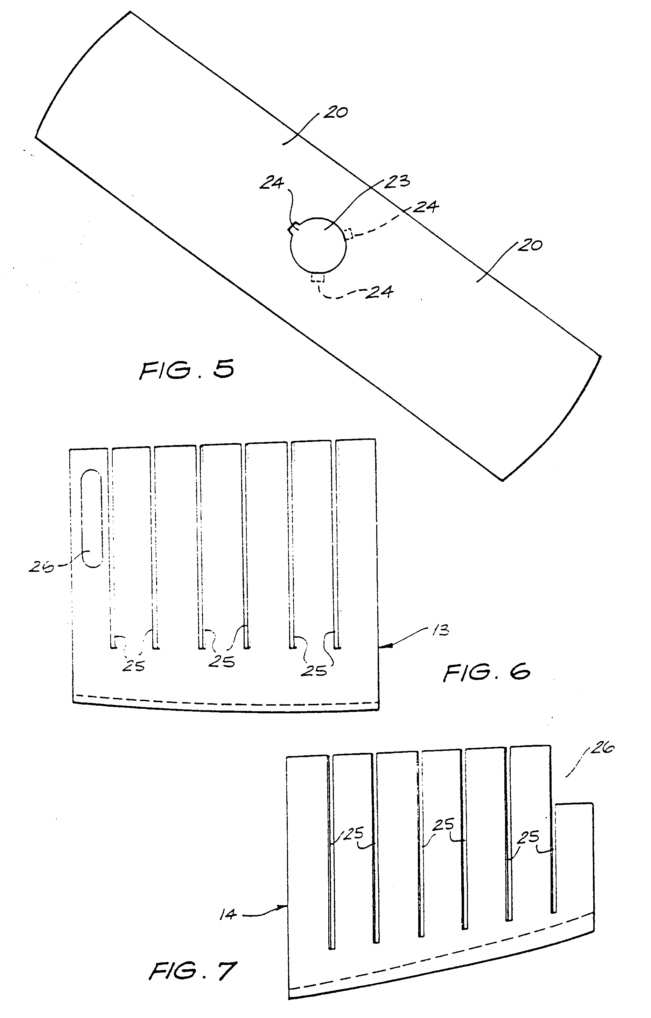

In Figures 1 to 7 there is depicted sharps destructor apparatus 10 comprising a generally cylindrical chamber 11, a cutter means 12, an upper cutter plate 13 and a lower cutter plate 14. The chamber 11 comprises an inlet 15 located above the cutter means 12, and an outlet 16 located below the cutter means 12. A retaining plate extends from a lower end of the upper outer plate 13 substantially to the roof of the container.

The cutter means 12 comprises a shaft 17 disposed generally parallel to the longitudinal axis of the cylindrical chamber 11, and rotatably mounted thereto by carrier bearing assemblies 18 which are fixed to the outside of the cylindrical chamber 11.

The shaft 17 projects to one side the cylindrical chamber 11 and has a pulley 19 fitted thereto to be driven by a vee belt drive from an electric motor (not shown) located with the housing 50.

A plurality of cutter blades 20 are mounted along the shaft 17 at predetermined intervals. The cutting blades 20 are fitted to and held in place by collars 21, and a square key provided on the shaft 17 engages key-ways 24 in the collars 21 and blades 20. The collars 21 and blades 20 are clamped together using a castellated nut 22 which is locked into position with split-pin 22a.

Each of the blades are provided with a central aperture 23 to receive the shaft 17, and a square key way 24 adapted to receive the square key of the shaft 17. There are provided three types of blades 20, each respectively having the key way 24 orientated approximately 120° angularly away from that of the other two blades 20. Each of the three types of blades 20 are positioned sequentially along the shaft so that each respective blade 20 extends in a direction 120° angularly displaced from the adjacent blade 20. The preferred embodiment depicted in the drawings is provided with six cutting blades 20. The blades 20 are approximately 10mm thick and are provided with a central groove 52 extending along the cutting edges 51.

The upper cutting plate 13 and lower cutting plate 14 are each provided with six slots 25 and when they are fitted in the chamber in the specified positions, the slots 25 are generally aligned with the cutting blades 20, which sweep and pass through the slots 25 when the shaft 17 is rotated.

The inlet 15 of the chamber 11 is provided proximate one end 11a and to one side 11b of the chamber 11. The upper cutter plate 13, in cross-section, is inclined downwards from the side 11b towards the central vertical plane of the chamber 11, and in longitudinal section, is inclined downwards from the end 11a to the other end 11c of the chamber.

The lower cutter plate 14, in cross-section, is inclined downwards from side 11b, and in longitudinal section, is inclined downwards from the other end 11c to the end 11a of the chamber.

The lower cutter plate 14 is aligned under the upper cutter plate 13. The lowermost portion of each of the upper cutter plate 13 and the lower cutter plate 14 define a recess 26 to allow the cut particles to fall therethrough due to gravity into a disposal bin.

Each of the cutter blades 20 cooperates with a respective slot 25 in each of the upper cutter plate 13 and lower cutter plate 14, whereby the width of the slots 25, and the clearance between the blades 20 and the slots 25 is predetermined so that the sharps entering the chamber 11 are generally comminuted due to impact and cutting action between the blades 20 and slots 25.

The chamber 11 is generally cylindrical and the shaft 17 is parallel to the walls of the chamber 11, and vertically aligned with the longitudinal axis of the chamber 11.

The shaft 17 is spaced from the bottom surface of the chamber 11 a distance slightly more than the radius of the blades 20. The internal diameter of the cylindrical chamber 11 is approximately one and one half times greater than the diameter of each blade 20.

Each cutter plate 13, 14 is downwardly inclined approximately 15° from the horizontal in a direction from the same side surface 11b of the chamber 11 to a point just past the central vertical plane 1 of the chamber 11. The shaft 7 rotates in a direction whereby the blades approach each of the cutter plates from above (as shown by arrow "A").

The longitudinal downward inclination of each of the cutter plates 13, 14 is approximately 10° from the horizontal. This allows larger cut particles to move across the plate (13 or 14) by gravity to the respective recesses 26.

In operation, the apparatus 10 reduces hypodermic syringes, needles and other small contaminated instruments such as scalpels blades, stitch cutters, by impact and cutting to a proportionately smaller volume, thus facilitating easier transportation, storage and subsequent disposal or incineration. The abovementioned materials are put into the apparatus 10 via the inlet 15, and exit therefrom via outlet 16 after the materials have passed along the upper and lower plates 13, 14.

The apparatus 10 reduces the volume of the material to be disposed of, and reduces the likelihood of physical contact with any of the contaminated materials.

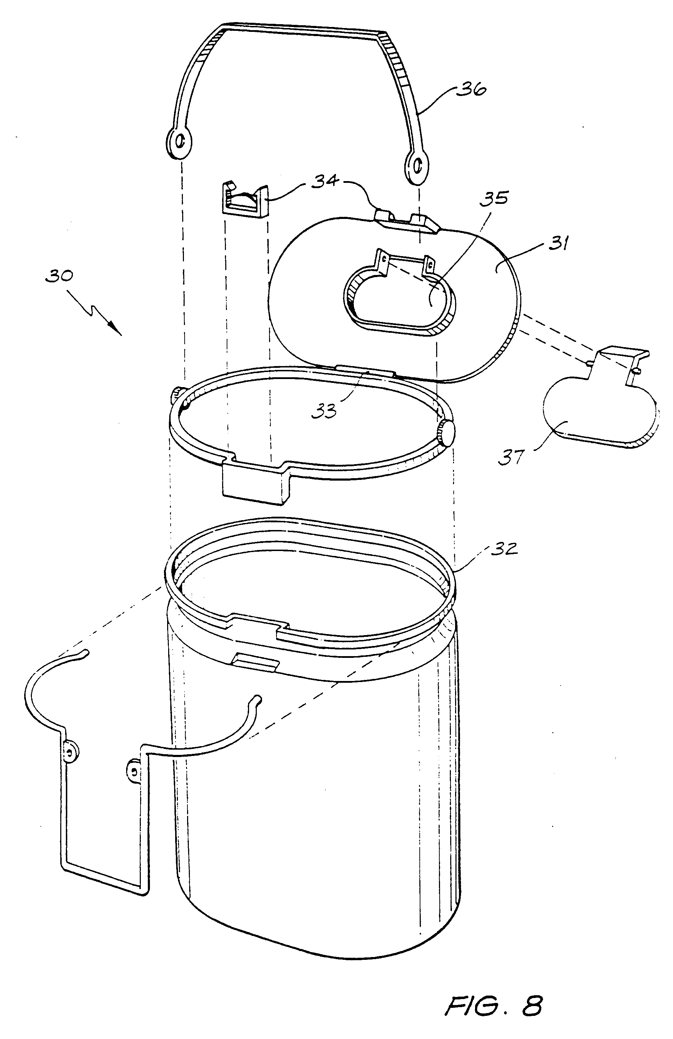

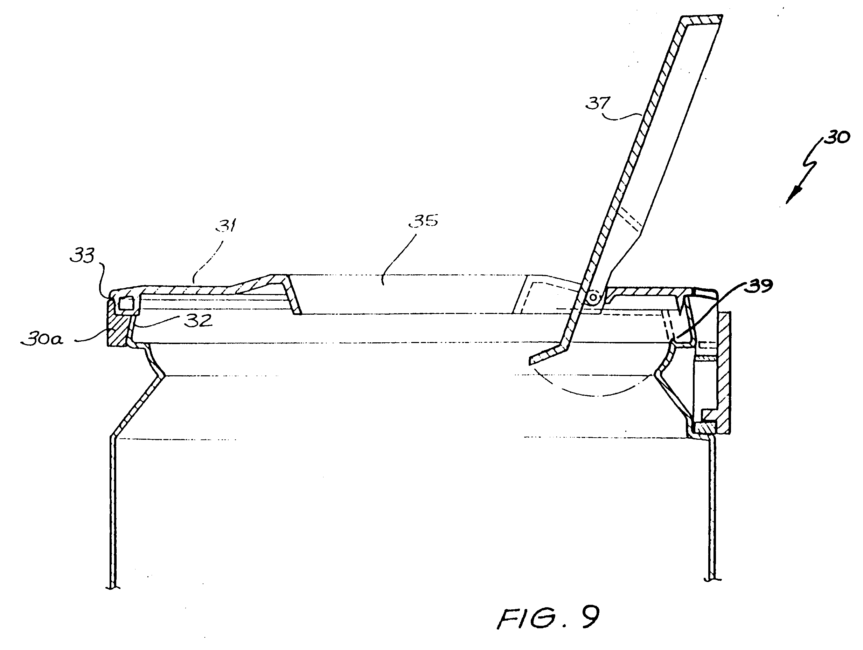

In Figures 8 and 9 there is depicted a sharps collection container 30 being a generally open-ended bucket-shape, with a pivotable lid 31 mounted to a top edge 32 of the container 30 with a hinge 33. The container 30 is provided with a latch mechanism 34 adapted to detachably retain the primary lid 31 in a closed position closing the open end of the container 30.

The primary lid 31 may be biased towards an open position extending transversely away from the plane of the opening, and towards the outside of the container 30.

The primary lid 31 is provided with a secondary lid 37 being pivotably mounted so as to be movable between a closed position closing an opening 35 in the primary lid 31, and an open position extending transversely away from the plane of the primary lid 31 outwards of the container 30. The secondary lid 37 is retained into the closed position by a resilient member 39. In the preferred embodiment the container 30 defines the resilient member 39. The retention may be overcome by additional force by the user to reopen the secondary lid 37.

The container 30 is further provided with a handle 36 for carrying purposes. The handle 36 is pivotable with respect to the container 30 between a retracted position and a carrying position. Alternatively or additionally, the container could be provided with a wall hanging member.

The container 30 also comprises an inwardly projecting ridge 32 on the inside surface thereof proximate the opening. This ridge generally prevents contaminated fluids from being discharged from the container if accidentally knocked over.

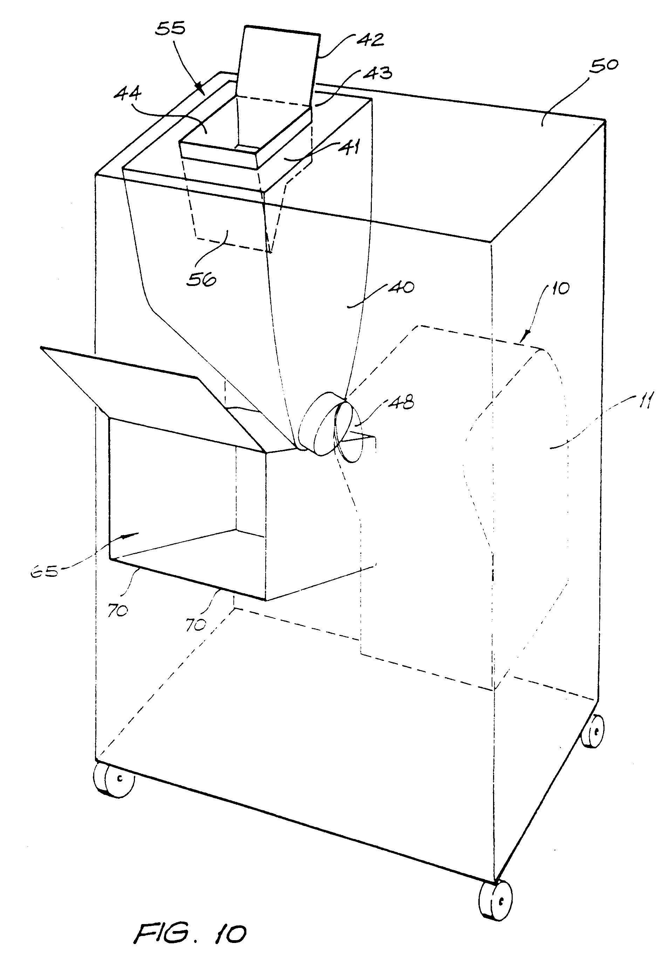

In Figure 10 there is depicted a bulk bin 40 within the housing 50 having a bottom opening 48 which communicates with the inlet 15 of the destructor apparatus 10.

The bulk bin portion 40 is provided with a lid 42 pivotably attached along a pivotal axis 43 on the housing 50. The lid 42 is pivotable between a closed position closing an upper opening 44 in the housing 50, and an open position allowing the container 30 to be inserted in the disposal receptacle 55.

The disposal receptacle 55 comprises an opening 44 communicating with the inside of the bin 40.

The receptacle 55 further comprises a locating means in the form of two slide guides 47a to locate an inverted collection container 30 in the receptacle 55. The lid 31 of the container 30 is caused to open when the container 30 is correctly positioned In the receptacle 55. That is, a latch release means 56 is provided in the receptacle 55 which is adapted to unlatch the latch mechanism 34, thereby allowing the lid 31 to move towards the open position and discharge the sharps directly into the bluk bin 40.

The bin 40 is also provided with a trap door 48 to control the discharge of the sharps into the inlet of the destructor apparatus 10. That is, the trap door 48 is operable so as to be openable only when the shaft 20 of the destructor apparatus 10 is rotating at the desired operating speed.

The bin 40 can also be provided with an agitator to maintain flow of the contaminated materials (sharps) through the bottom opening 48.

In use, the container 30 is used to collect the contaminated sharps by insertion through the secondary lid 37. When the container is full the secondary lid 37 is closed and the container may then be emptied into the bin 40 by inverting the container 30 and moving it into position in the receptacle 55 using the locating means 47. The latch release means 56 unlatches the latch mechanism 34 to release the contaminated sharps into the bulk bin 40 due to gravity. A number of container loads of sharps may be deposited and stored in the bin 40 until it is desired to destroy and dispose of the contaminated sharps. The destructor apparatus 10 can then be operated by driving the shaft 20, and opening the trap door 48 of the bulk bin 40 whereby the sharps are caused to fall through the inlet 15 into the chamber 11. The sharps are then reduced by impact and cutting and discharged through the outlet 16.

The material being discharged through the outlet 16 is generally safe, that is, the sharps are comminuted and do not pose a danger to the personnel who handle the material.

The comminuted contaminated materials are fed downwards through the chamber 11 to the bottom outlet 16, and collected in a sealable disposal bin for subsequent disposal. The bins may be disposed of by either incineration or burial at local garbage tips.

The housing 50 further comprises a washing receptable 65 adapted to slidably receive the container. The washing receptacle is similar to the discharge receptable 55 in that a projection 66 is provided which is disposed to unlatch the latch means of the container thereby allowing the primary lid to open. Washing jets 67 are provided which, selectively, direct sanitising fluid into the container. The washed container is then ready for reuse without risk of contaminated fluid being discharged therefrom when moved away from the housing 50.

Sanitising fluid is also Introduced into the sharps either when they are being discharged from the container into the bulk bin 40, in the bulk bin 40 or within the chamber 11. This renders the comminuted material environmentally safe.

The comminuted material discharged from the apparatus 10 is able to be buried or incinerated. Previously, whole syringes were incinerated which took considerable time and energy, and was incomplete. That is, incineration leaves a relatively large volume of ashes. When the comminuted material of the present invention is incinerated, there is no residue and power requirements are substantially reduced.

The above described disposal apparatus is easy to use, and attends to the safety of staff and personnel whose job it is to handle the contaminated wastes on a daily basis.

## Claims

1. A reusable sharps container (30) for receiving sharps, the container having a primary opening and a primary lid (31) pivotably mounted to said container (30) and movable with respect to the container (30) between a closed position closing the primary opening, and an open position opening the primary opening; characterised by
said container (30) further having a secondary opening (35) in said primary lid (31) and a secondary lid (37) pivotably mounted to said primary lid (31) and movable between a closed position closing the secondary opening (35), and an open position opening the secondary opening (35);
said container (30) further comprising a latch means (34) arranged to retain the primary lid (31) in the closed position, in normal use, and adapted to automatically open the primary lid (31) when said latch means (34) is engaged by a latch opening means of a sharps destructor apparatus,
the secondary lid (37) being movable, in normal use, to open the secondary opening (35) whereby sharps are insertable into the container (30) through said secondary opening (35), the primary lid (31) not being movable from its closed position, in normal use, but only when the latch means (34) is engaged by the latch opening means (56) to open the primary opening whereby the sharps are removable through the primary opening to empty the container (30) so that the container can be reused.

2. The container (30) of claim 1, further comprising a ridge (32) provided on an inside surface thereof generally coextensive with and proximate the primary opening.

3. The container (30) of either claim 1 or claim 2, wherein said secondary lid (37) is detachably retainable in said closed position by a resilient member (39).

4. The container (30) of any one of claims 1 to 3, wherein said latch means (34) comprises a striker member mounted on the primary lid (31) or the container (30), and a latch mounted on said container (30) or said primary lid (31) and adapted to releasably retain the striker member.

5. The container (30) of claim 4 wherein said latch comprises a hook portion adapted to resiliently deform and thereby retain said striker member.

6. The container (30) of any one of claims 1 to 5 further comprising a carry handle (36).

7. The container (30) of any one of claims 1 to 6 further comprising a wall hanging member adapted to mount the container to a vertical support.

8. A combination of the container (30) of any one of claims 1 to 7 and a sharps destructor apparatus (10) having a disposal receptacle (55) for receiving the container (30).

9. The combination of claim 8, wherein a latch opening means (56) is provided associated with the disposal receptacle to engage said latch means (34) to re-open the primary lid (31) only when the primary opening of the container (30) is brought within said disposal receptacle (55) of said apparatus.

10. The combination of claim 9, wherein said latch opening means (56) comprises a generally straight projection.

11. The combination of claim 10, wherein said sharps destructor apparatus comprises:
a chamber (11);
a driven rotating cutter (12) comprising a shaft (17) rotatably mounted in said chamber (11) and having a plurality of cutter blades (20) mounted at predetermined positions along said shaft (17) so as to rotate therewith and thereby cut sharps within the chamber (11);
at least one cutter plate (13, 14) mounted in said chamber (11) and having a plurality of elongate slots (25) of predetermined width, said cutter blades (20) being generally aligned with respective slots (25) in said plate or plates (13, 14);
an inlet (15) located above said plate or plates (13, 14);
and an outlet (16) located below said plate or plates (13, 14);
said plate or plates (13, 14) being inclined downwardly from the inlet (15) towards the outlet (16).

12. The combination of claim 11 further comprising a washing means (65) adapted to wash the container (30) and/or the sharps with a sanitising fluid.

13. The combination of either claim 11 or 12, comprising two cutter plates (13, 14), one plate (13) being generally superposed over the other plate (14), each of said plates (13, 14) being downwardly inclined longitudinally of the chamber and defining a discharge opening at a lower end;
said inlet (15) being generally above an upper end of said one plate (13);
an upper end of said other plate (14) being generally below the discharge opening of said one plate;
and said outlet (16) being generally below said discharge opening of said other plate (14).

14. The combination of claim 13, wherein each of said plates (13, 14) is also downwardly inclined and extends from a side of said chamber (11) at least to a central longitudinal vertical plane of the chamber (11).

15. The combination of claim 14, further comprising a retaining plate extending upwardly from a lower side of the one plate (13) substantially to the roof of the chamber (11).

16. The combination of any one of claims 11 to 15, wherein said blades (20) are approximately 10mm thick.

17. The combination of any one of claims 11 to 16, wherein said disposal receptacle (55) is in direct communication with said chamber (11).

18. The combination of any one of claims 11 to 17, wherein said blades (20) are provided with one or more grooves (52) on their cutting edges.

19. The combination of any one of claims 13 to 17, wherein the longitudinal downward inclination of each of said plates (13, 14) is approximately 10° from the horizontal.

20. The combination of claim 19 wherein the downward inclination of each of said plates (13, 14) from a side of said chamber (11) is approximately 15° from the horizontal.

21. A method of disposing of contaminated medical tools, sharps and other materials, characterized by:
closing a primary opening in a reusable container (30) with a primary lid (31) which is secured in a closed position by a latch means (34);
collecting said tools, sharps, and other materials in said reusable container (30) by inserting same through a secondary opening (35) in said primary lid (31) of said container (30), the secondary opening (35) being closable by a secondary lid (37);
inverting and inserting said reusable container (30) in a receptacle (55) of an apparatus which is adapted to automatically release said latch means;
whereby said primary lid (31) opens and said tools, sharps, and other materials are discharged directly into said receptacle (55) of said apparatus; and
removing the reusable container (30) from the receptacle (55) for reuse.

22. The method of claim 21 wherein said apparatus is a destructor apparatus (10) adapted to comminute said material by impact and cutting action.

23. The method of either claim 21 or claim 22 wherein said materials and said container (30) are washed by a sanitising fluid.

24. The method of either claim 22 or claim 23 wherein said materials are introduced into a chamber (11) of said destructor apparatus (10) through an inlet (15) onto a cutting plate (13) which is downwardly inclined longitudinally of the chamber (11), the plate (13) having a plurality of slots (25) aligned with a plurality of blades (20) mounted on a rotatable shaft (17), said blades (20) and said plate (13) cooperating to cut or impact the materials which move down along the plate (13) to a discharge opening at the lower end of the plate (13) and onto another cutting plate (14) and through an outlet (16) of said chamber (11) in a generally comminuted state.

25. The method of any one of claims 22 to 24 wherein said comminuted material is discharged into a disposal bin.

26. The method of any one of claims 21 to 25, wherein the materials are cut using cutting edges formed on the blades (20).

## Patentansprüche

1. Wiederverwendbarer Behälter (30) zur Aufnahme von scharfen Gegenständen, wobei der Behälter eine primäre Öffnung und einen primären Verschlußdeckel (31) aufweist, der drehbar an dem Behälter (30) befestigt ist und der bezüglich des Behälters (30) zwischen einer verschlossenen Stellung, in der die primäre Öffnung verschlossen ist, und einer geöffeneten Stellung, in der die primäre Öffnung geöffnet ist, bewegbar ist,
**dadurch gekennzeichnet,**
daß der Behälter (30) eine sekundären Öffnung (35) aufweist, die in dem primären Verschlußdeckel (31) angeordnet ist, und einen sekundären Verschlußdeckel (37), der drehbar an dem primären Verschlußdeckel (31) befestigt ist und zwischen einer verschlossenen Stellung, in der die sekundäre Öffnung (35) verschlossen ist, und einer geöffneten Stellung, in der die sekundäre Öffnung (35) geöffnet ist, bewegbar ist;
daß der Behälter (30) eine Sperreinrichtung (34) aufweist, mittels derer der primäre Verschlußdeckel (31) im Normalbetrieb in der verschlossenen Stellung zurückhaltbar ist und mittels derer der primäre Verschlußdeckel (31) automatisch geöffnet werden kann, wenn die Sperreinrichtung (34) durch eine Entriegelungseinrichtung einer Vorrichtung zur Vernichtung scharfer Gegenstände angegriffen wird, und
daß der sekundäre Verschlußdeckel (37) im Normalbetrieb bewegbar ist, um die sekundäre Öffnung (35) zu öffnen, wodurch scharfe Gegenstände in den Behälter (30) durch die sekundäre Öffnung (35) einführbar sind, wobei der primäre Verschlußdeckel (31) im Normalbetrieb nicht aus seiner verschlossenen Stellung bewegbar ist, sondern nur, wenn die Sperreinrichtung (34) durch die Entriegelungseinrichtung (56) angegriffen wird, um die primäre Öffnung zu öffnen, wodurch die scharfen Gegenstände durch die primäre Öffnung entfernbar sind, um den Behälter (30) zu entleeren, so daß der Behälter wiederverwendbar ist.

2. Behälter (30) nach Anspruch 1, **dadurch gekennzeichnet**, daß der Behälter einen vorstehenden Rand (32) aufweist, der an dessen Innenseite angeordnet ist und der diesen im wesentlichen umfaßt und der in der Nähe der primären Öffnung angeordnet ist.

3. Behälter (30) nach Anspruch 1 oder 2, **dadurch gekennzeichnet**, daß der sekundäre Verschlußdeckel (37) abnehmbar ist und in der verschlossenen Stellung durch ein federndes Bauteil (39) zurückhaltbar ist.

4. Behälter (30) nach einem der Ansprüche 1 bis 3, **dadurch gekennzeichnet**, daß die Sperreinrichtung (34) ein bolzenförmiges Bauteil aufweist, das auf dem primären Verschlußdeckel (31) oder dem Behälter (30) angeordnet ist, wobei eine Sperre an dem Behälter (30) oder dem primären Verschlußdeckel (31) angeordnet ist, mittels derer das bolzenförmige Bauteil freigebbar zurückgehalten werden kann.

5. Behälter (30) nach Anspruch 4, **dadurch gekennzeichnet**, daß die Sperre einen hakenförmigen Bereich aufweist, mittels dessen das bolzenförmige Bauteil federnd deformierbar und dadurch zurückhaltbar ist.

6. Behälter (30) nach einem der Ansprüche 1 bis 5, **dadurch gekennzeichnet**, daß ein Handgriff (36) vorgesehen ist.

7. Behälter nach einem der Ansprüche 1 bis 6, **dadurch gekennzeichnet**, daß eine Wandaufhängevorrichtung vorgesehen ist, mittels derer der Behälter an einem vertikal angeordneten Träger befestigbar ist.

8. Anordnung eines Behälters (30) nach einem der Ansprüche 1 bis 7 zusammen mit einer Vorrichtung (10) zur Vernichtung scharfer Gegenstände, die eine Abfallaufnahmeeinrichtung (55) zur Aufnahme des Behälters (30) aufweist.

9. Anordnung nach Anspruch 8, **dadurch gekennzeichnet**, daß eine Entriegelungsvorrichtung (56) vorgesehen ist, die mit der Abfallaufnahmeeinrichtung verbunden ist, um an die Sperreinrichtung (34) anzugreifen, um den primären Verschlußdeckel (31) erneut nur dann zu öffnen, wenn die primäre Öffnung des Behälters (30) in die Abfallaufnahmeeinrichtung (55) der Vorrichtung eingebracht ist.

10. Anordnung nach Anspruch 9, **dadurch gekennzeichnet**, daß die Entriegelungseinrichtung (56) einen im wesentlichen geradlinigen Vorsprung aufweist.

11. Anordnung nach Anspruch 10, **dadurch gekennzeichnet**, daß die Vorrichtung zur Vernichtung scharfer Gegenstände aufweist:
eine Kammer (11),
eine angetriebene rotierende Schneidvorrichtung (12), welche eine rotierbar in der Kammer (11) angeordnete Welle und eine Mehrzahl von Schneidmessern (20) aufweist, die an vorbestimmten Positionen entlang der Welle (17) angeordnet sind, so daß diese mit der Welle drehbar sind und dadurch scharfe Gegenstände innerhalb der Kammer (11) zerteilten;
mindestens eine Schneideplatte (13, 14), die innerhalb der Kammer (11) befestigt ist und eine Mehrzahl von länglichen Schlitzen (25) mit vorbestimmter Weite aufweist, wobei die Schneidmesser (20) im wesentlichen entlang der entsprechenden Schlitze (25) in der Platte oder in den Platten (13, 14) ausgerichtet sind;
eine Einführöffnung (15), die oberhalb der Platte oder den Platten (13, 14) angeordnet sind;
eine Austrittsöffnung (16), die unterhalb der Platte oder den Platten (13, 14) angeordnet ist,
wobei die Platte oder die Platten (13, 14) von der Einführöffnung (15) abwärts geneigt sind in Richtung auf die Austrittsöffnung (16).

12. Anordnung nach Anspruch 11, **dadurch gekennzeichnet**, daß eine Wascheinrichtung (65) vorgesehen ist, mittels derer der Behälter (30) und/oder die scharfen Gegenstände mit einer Desinfektionsflüssigkeit waschbar sind.

13. Anordnung nach einem der Ansprüche 11 oder 12, **dadurch gekennzeichnet**, daß zwei Schneidplatten (13, 14) vorgesehen sind, wobei eine der Platten (13) im wesentlichen oberhalb der anderen Platte (14) angeordnet ist, wobei jede der Platten (13, 14) längs der Kammer abwärts geneigt angeordnet sind und an einem unteren Ende eine Entleerungsöffnung begrenzen;
daß die Einführöffnung (15) im wesentlichen oberhalb des oberen Endes der einen Platte (13) angeordnet ist;
daß ein oberes Ende der anderen Platte (14) im wesentlichen unterhalb der Entleerungsöffnung der einen Platte angeordnet ist;
daß die Austrittsöffnung (16) im wesentlichen unterhalb der Entleerungsöffnung der anderen Platte (14) angeordnet ist.

14. Anordnung nach Anspruch 13, **dadurch gekennzeichnet**, daß jede der Platten (13, 14) abwärts geneigt angeordnet ist und sich von einer Seite der Kammer (11) zumindest bis auf eine zentrale längs verlaufende Vertikalebene der Kammer (11) erstreckt.

15. Anordnung nach Anspruch 14, **dadurch gekennzeichnet**, daß eine Rückhalteplatte vorgesehen ist, die sich von einem unteren Ende der einen Platte (13) aufwärts nahezu bis an das Dach der Kammer (11) erstreckt.

16. Anordnung nach einem der Ansprüche 11 bis 15, **dadurch gekennzeichnet**, daß die Messer (20) eine Dicke von ca. 10 mm aufweisen.

17. Anordnung nach einem der Ansprüche 11 bis 16, **dadurch gekennzeichnet,** daß die Abfallaufnahmeeinrichtung (55) in direkter Verbindung mit der Kammer (11) steht.

18. Anordnung nach einem der Ansprüche 11 bis 17, **dadurch gekennzeichnet**, daß eine oder mehrere Einschnitte (52) auf den Schneidkanten der Messer (20) vorgesehen sind.

19. Anordnung nach einem der Ansprüche 13 bis 17, **dadurch gekennzeichnet**, daß die longitudinale Abwärtsneigung einer jeden der Platten (13, 14) ungefähr 10° aus der Horizontalen beträgt.

20. Anordnung nach Anspruch 19, **dadurch gekennzeichnet**, daß die Abwärtsneigung einer jeden der Platten (13, 14) von einer Seite der Kammer (11) ungefähr 15° aus der Horizontalen beträgt.

21. Verfahren zu Beseitigung von kontaminierten medizinischen Geräten, scharfen Gegenständen und anderen Stoffe, **dadurch gekennzeichnet**,
daß eine primäre Öffnung in einem wiederverwendbaren Behälter (30) mittels eines primären Verschlußdeckels (31) verschlossen wird, welcher in einer geschlossenen Stellung mittels einer Sperreinrichtung (34) gesichert ist;
daß die Geräte, scharfen Gegenstände und anderen Stoffe in dem wiederverwendbaren Behälter (30) gesammelt werden, indem diese durch eine im primären Verschlußdeckel (31) des Behälters (30) angeordnete sekundäre Öffnung (35) eingeführt werden, wobei die sekundäre Öffnung (35) mittels eines sekundären Verschlußdeckels (37) verschließbar ist;
daß der wiederverwendbare Behälter (30) umgedreht wird und in einer Aufnahmeeinrichtung (55) einer Vorrichtung eingeführt wird, mittels welcher die Sperreinrichtung automatisch entriegelbar ist;
wodurch der primäre Verschlußdeckel (31) geöffnet wird und die Geräte, scharfen Gegenstände und anderen Stoffe direkt in die Aufnahmeeinrichtung (55) der Vorrichtung entleert werden; und
der wiederverwendbare Behälter (30) von der Aufnahmeeinrichtung (55) zur Wiederverwendung entfernt wird.

22. Verfahren nach Anspruch 21, **dadurch gekennzeichnet**, daß die Vorrichtung eine Vernichtungsvorrichtung (10) darstellt, mittels derer die Stoffe durch Schlag- und Schneidevorgänge zerkleinert werden.

23. Verfahren nach Anspruch 21 oder 22, **dadurch gekennzeichnet**, daß die Stoffe und der Behälter (30) mit einer Desinfektionsflüssigkeit gewaschen werden.

24. Verfahren nach Anspruch 22 oder 23, **dadurch gekennzeichnet**, daß die Stoffe in eine Kammer (11) der Vernichtungsvorrichtung (10) durch eine Einführöffnung (15) auf eine Schneidplatte (13) eingebracht werden, welche längs der Kammer (11) abwärts geneigt angeordnet ist, wobei die Platte (13) eine Mehrzahl von Schlitzen (25) aufweist, die mit einer Mehrzahl von auf einer rotierbaren Welle (17) befestigten Messern (20) ausgerichtet sind, wobei die Messer (20) und die Platte (13) zusammenwirken, um die Stoffe zu schneiden oder zu zerschlagen, welches sich entlang der Platte (13) abwärts bewegen in Richtung auf eine am unteren Ende der Platte (13) angeordnete Entleerungsöffnung, auf eine weitere Schneidplatte (14) und in einem im wesentlichen zerkleinerten Zustand durch eine Austrittsöffnung (16) der Kammer (11).

25. Verfahren nach einem der Ansprüche 22 bis 24, **dadurch gekennzeichnet**, daß die zerkleinerten Stoff ein einen Abfallbehälter entleert wird.

26. Verfahren nach einem der Ansprüche 21 bis 25, **dadurch gekennzeichnet**, daß die Stoffe mittels auf den Messern (20) angeordneter Schneidkanten zerkleinert wird.

## Revendications

1. Conteneur d'objets tranchants réutilisable (30) destiné à recevoir des objets tranchants, ce conteneur comportant une ouverture primaire et un couvercle primaire (31) monté en pivotement sur le conteneur (30) et pouvant se déplacer par rapport à ce conteneur (30) entre une position de fermeture fermant l'ouverture primaire, et une position d'ouverture ouvrant l'ouverture primaire ; caractérisé en ce que
le conteneur (30) comporte en outre une ouverture secondaire (35) formée dans le couvercle primaire (31) et un couvercle secondaire (37) monté en pivotement sur le couvercle primaire (31) et pouvant se déplacer entre une position de fermeture fermant l'ouverture secondaire (35), et une position d'ouverture ouvrant l'ouverture secondaire (35) ;
le conteneur (30) comprend en outre un dispositif de loquet (34) disposé pour retenir le couvercle primaire (31) dans la position de fermeture, en cours d'utilisation normale, et conçu pour ouvrir automatiquement le couvercle primaire (31) lorsque ce dispositif de loquet (34) est enclenché par un dispositif d'ouverture de loquet d'un appareil de destruction d'objets tranchants ;
le couvercle secondaire (37) est manoeuvrable, en cours d'utilisation normale, pour ouvrir l'ouverture secondaire (35) de façon qu'on puisse introduire des objets tranchants dans le conteneur (30) par l'ouverture secondaire (35), le couvercle primaire (31) n'étant pas mobile à partir de sa position de fermeture, en cours d'utilisation normale, mais ne pouvant être manoeuvré que lorsque le dispositif de loquet (34) est enclenché par le dispositif d'ouverture de loquet (56) pour ouvrir l'ouverture primaire de façon qu'on puisse retirer les objets tranchants par cette ouverture primaire afin de vider le conteneur (30) pour pouvoir ensuite le réutiliser.

2. Conteneur (30) selon la revendication 1, caractérisé en ce qu'il comprend en outre une saillie (32) formée sur sa surface intérieure et s'étendant d'une façon générale tout autour et à proximité de l'ouverture primaire.

3. Conteneur (30) selon l'une des revendications 1 ou 2, caractérisé en ce que le couvercle secondaire (37) est retenu de façon détachable dans sa position de fermeture par un élément élastique (39).

4. Conteneur (30) selon l'une quelconque des revendications 1 à 3, caractérisé en ce que le dispositif de loquet (34) comprend un élément de butoir monté sur le couvercle primaire (31) ou sur le conteneur (30), et un loquet monté sur le conteneur (30) ou sur le couvercle primaire (31), ce loquet étant destiné à retenir de façon détachable l'élément de butoir.

5. Conteneur (30) selon la revendication 4, caractérisé en ce que le loquet comprend une partie de crochet destinée à se déformer élastiquement et à retenir ainsi l'élément de butoir.

6. Conteneur (30) selon l'une quelconque des revendications 1 à 5, caractérisé en ce qu'il comprend en outre une poignée de transport (36).

7. Conteneur (30) selon l'une quelconque des revendications 1 à 6, caractérisé en ce qu'il comprend en outre un élément d'accrochage à une paroi, destiné à monter le conteneur sur un support vertical.

8. Combinaison du conteneur (30) selon l'une quelconque des revendications 1 à 7, avec un appareil de destruction d'objets tranchants (10) comportant un récipient de mise au rebut (55) pour recevoir le conteneur (30).

9. Combinaison selon la revendication 8, caractérisée en ce qu'un dispositif d'ouverture de loquet (56) est associé au récipient de mise au rebut de manière à enclencher le dispositif de loquet (34) pour ne rouvrir le couvercle primaire (31) que lorsque l'ouverture primaire du conteneur (30) est amenée à l'intérieur du récipient de mise au rebut (55) de l'appareil.

10. Combinaison selon la revendication 9, caractérisée en ce que le dispositif d'ouverture de loquet (56) comprend une projection généralement droite.

11. Combinaison selon la revendication 10, caractérisée en ce que l'appareil de destruction d'objets tranchants comprend :
une chambre (11) ;
un couteau rotatif entraîné (12) comprenant un arbre (17) monté en rotation dans la chambre (11) et comportant une pluralité de lames de couteau (20) montées dans des positions prédéterminées le long de l'arbre (17) de manière à tourner avec celui-ci et à couper ainsi les objets tranchants à l'intérieur de la chambre (11) ;
au moins une plaque de couteau (13, 14) montée dans la chambre (11) et comportant une pluralité de fentes allongées (25) de largeur prédéterminée, les lames de couteau (20) étant généralement alignées avec des fentes respectives (25) de la plaque ou des plaques (13, 14) ;
une entrée (15) placée au-dessus de la plaque ou des plaques (13, 14) ;
et une sortie (16) placée au-dessous de la plaque ou des plaques (13, 14) ;
la plaque ou les plaques (13, 14) étant inclinées vers le bas à partir de l'entrée (15) vers la sortie (16).

12. Combinaison selon la revendication 11, caractérisée en ce qu'elle comprend en outre un dispositif de lavage (65) destiné à laver le conteneur (30) et/ou les déchets tranchants, au moyen d'un fluide sanitaire.

13. Combinaison selon l'une des revendications 11 ou 12, caractérisée en ce qu'elle comprend deux plaques de couteau (13, 14), une plaque (13) étant généralement superposée au-dessus de l'autre plaque (14), chacune de ces plaques (13, 14) étant inclinée vers le bas dans le sens longitudinal de la chambre et définissant une ouverture de décharge à une extrémité inférieure ;
l'entrée (15) étant généralement au-dessus d'une extrémité supérieure de la plaque (13) ;
une extrémité supérieure de l'autre plaque (14) étant généralement au-dessous de l'ouverture de décharge de la première plaque ;
et la sortie (16) étant généralement au-dessous de l'ouverture de décharge de l'autre plaque (14).

14. Combinaison selon la revendication 13, caractérisée en ce que chacune des plaques (13, 14) est également inclinée vers le bas et part d'un côté de la chambre (11) pour aller au moins jusqu'à un plan vertical longitudinal central de la chambre (11).

15. Combinaison selon la revendication 14, caractérisée en ce qu'elle comprend en outre une plaque de retenue s'étendant vers le haut à partir d'un côté inférieur d'une plaque (13), pour aller essentiellement jusqu'au sommet de la chambre (11).

16. Combinaison selon l'une quelconque des revendications 11 à 15, caractérisée en ce que les lames (20) ont une épaisseur d'environ 10 mm.

17. Combinaison selon l'une quelconque des revendications 11 à 16, caractérisée en ce que le récipient de mise au rebut (55) est en communication directe avec la chambre (11).

18. Combinaison selon l'une quelconque des revendications 11 à 17, caractérisée en ce que les lames (20) sont munies d'une ou plusieurs rainures (52) sur leurs bords de coupe.

19. Combinaison selon l'une quelconque des revendications 13 à 17, caractérisée en ce que l'inclinaison longitudinale vers le bas de chacune des plaques (13, 14) est d'approximativement 10° par rapport à l'horizontale.

20. Combinaison selon la revendication 19, caractérisée en ce que l'inclinaison vers le bas de chacune des plaques (13, 14) à partir d'un côté de la chambre (11), est d'approximativement 15° par rapport à l'horizontale.

21. Procédé de mise au rebut d'instruments médicaux, d'objets tranchants et autres matériaux contaminés, caractérisé en ce qu'il consiste à :
fermer une ouverture primaire d'un conteneur réutilisable (30) au moyen d'un couvercle primaire (31) fixé dans une position de fermeture par un dispositif de loquet (34) ;
rassembler les instruments, objets tranchants et autres matériaux dans le conteneur réutilisable (30) en les introduisant par une ouverture secondaire (35) formée dans le couvercle primaire (31) du conteneur (30), cette ouverture secondaire (35) pouvant être fermée par un couvercle secondaire (37) ;
retourner à l'envers et introduire le conteneur réutilisable (30) dans un récipient (55) d'un appareil conçu pour ouvrir automatiquement le dispositif de loquet ;
ce qui permet ainsi au couvercle primaire (31) de s'ouvrir et aux instruments, objets tranchants et autres matériaux, d'être déchargés directement dans le récipient (55) de l'appareil ; et
retirer le conteneur réutilisable (30) du récipient (55) afin de le réutiliser.

22. Procédé selon la revendication 21, caractérisé en ce que l'appareil est un appareil de destruction (10) destiné à concasser les matériaux par une action de chocs et de coupe.

23. Procédé selon l'une ou l'autre des revendications 21 et 22, caractérisé en ce que les matériaux et le conteneur (30) sont lavés au moyen d'un fluide sanitaire.

24. Procédé selon l'une ou l'autre des revendications 22 et 23, caractérisé en ce que les matériaux sont introduits dans une chambre (11) de l'appareil de destruction (10) en passant par une entrée (15) pour tomber sur une plaque de coupe (13) inclinée vers le bas dans le sens longitudinal de la chambre 11, la plaque (13) comportant une pluralité de fentes (25) éloignées, avec une pluralité de lames (20) montées sur un arbre rotatif (17), les lames (20) et la plaque (13) coopérant pour couper ou briser les matériaux qui descendent le long de la plaque (13) vers une ouverture de décharge située à l'extrémité inférieure de la plaque (13), et tombent sur une autre plaque de coupe (14) puis passent à travers une ouverture de sortie (16) de la chambre (11) dans un état généralement concassé en petits morceaux.

25. Procédé selon l'une quelconque des revendications 22 à 24, caractérisé en ce que le matériau concassé est déchargé dans une boîte à jeter.

26. Procédé selon l'une quelconque des revendications 21 à 25, caractérisé en ce que les matériaux sont coupés en utilisant les bords de coupe formés sur les lames (20).
